(19) Europäisches Patentamt
European Patent Office
Office européen des brevets

(11) EP 4 721 724 A1

(12) **EUROPEAN PATENT APPLICATION**

(43) Date of publication:
**08.04.2026 Bulletin 2026/15**

(21) Application number: **24203826.3**

(22) Date of filing: **01.10.2024**

(51) International Patent Classification (IPC):
***A61K 8/42*** *(2006.01)* ***A61K 8/898*** *(2006.01)*
***A61Q 5/00*** *(2006.01)* ***A61Q 5/12*** *(2006.01)*

(52) Cooperative Patent Classification (CPC):
**A61Q 5/002; A61K 8/42; A61K 8/898;** A61Q 5/02;
A61Q 5/12

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH MA MD TN**

(71) Applicant: **Beautyge, S.L.**
**08940 Cornella de Llobregat (ES)**

(72) Inventors:
- **COLOM VIVES, Olga**
**08940 Cornella de Llobregat (Barcelona) (ES)**
- **VALLECILLOS LÓPEZ, Rocío**
**08940 Cornella de Llobregat (Barcelona) (ES)**
- **BEATO MERINO, Raquel**
**08940 Cornella de Llobregat (Barcelona) (ES)**

(74) Representative: **Gallardo, Antonio M.**
**Revlon**
**WTC Almeda Park**
**Tirso de Molina 40, Ed. 4**
**08940 Cornellà de Llobregat (Barcelona) (ES)**

# (54) METHOD FOR INCREASING THE VOLUME OF KERATIN FIBERS

(57) A method for increasing the volume of keratin fibers is described. The method for increasing the volume of keratin fibers comprises the steps of:
(i) treating the keratin fibers by topical application of a cosmetic composition comprising, in a cosmetically acceptable medium:
(c) at least urea and/or an urea derivative selected from N,N-dimethylurea, N-(2-hydroxyethyl)urea, N-(3-hydroxypropyl)urea, N-(2-hydroxypropyl)urea, N-(2-hydroxy-2-methyl-prop-2-yl)urea, N-(1,3-dihydroxy-2-methyl-prop-2-yl)urea, N-(1,3-dihydroxy-2-hydroxymethyl-prop-2-yl)urea, and mixtures thereof; and
(d) at least one film-forming amino-silicone polymer selected from Polysilicone-35, Polysilicone-33, Polysilicone-29, and mixtures thereof;

(ii) allowing the composition to remain in contact with the keratin fibers for a period ranging from about 5 seconds to about 96 hours.

This method increases the volume of keratin fibers, in particular human hair, without leaving the hair stiff or sticky, without leaving substantial adhesion between the hair fibers while maintaining the improvement in the volume of hair after washing it with a shampoo.

EP 4 721 724 A1

## Description

### TECHNICAL FIELD

A method for increasing the volume of keratin fibers is described. This method increases the volume of keratin fibers, in particular human hair, without leaving the hair stiff or sticky, without leaving substantial adhesion between the hair fibers while maintaining the improvement in the volume of hair after washing it with a shampoo.

### BACKGROUND

The information provided below is not admitted to be prior art to the present disclosure, but is provided solely to assist the understanding of the reader.

Consumers with fine or thin hair often desire more hair volume, which is the visible bulkiness of hair, while controlling undesirable fly-away of hair. Fly-away hair is due to the increased level of static (i.e., frizzy hair), and represents volume increase of only very minor amount of the hair as a whole. In addition, fly-away hair is more difficult to comb, requiring increased force to comb the hair, which in turn can wear the outer surface of the hair and cause cracks and breaks in the hair.

Other concept related to hair health is hair density. Hair density refers to the number of strands per square inch in the scalp. One of the main differences between hair density and volume is that density refers to the amount of hair, while volume refers to the space that hair occupies. A person with a lot of volume may have little density, and another with greater density may have little volume in cases, especially with straight hair.

One approach towards increasing hair volume is to use products such as hair styling gels and mousses. During their wet state on hair, gels and mousses help in the creation of a hair style. On drying, a polymeric film forms on the hair, which helps in holding and maintaining the created hair volume. However, this effect is often short lived and may be at least partially lost by combing the hair thoroughly. Also, some of the polymers used in these products can leave the hair feeling stiff, tacky or rough.

Other techniques used to increase the appearance of hair volume include perming, back combing, and use of high temperature heated implements such as straightening irons. However, all these processes can cause a degree of mechanical or chemical damage to the hair if used excessively.

Therefore, there is a need for hair treatments that can provide an increase (improvement) in the volume of hair without the negatives associated with the prior art methods described above. In particular, there is a need for hair treatments that can increase the volume of hair without leaving the hair stiff or sticky, without leaving substantial adhesion between the hair fibers while maintaining the improvement in the volume of hair after washing it with a shampoo.

### SUMMARY

Described herein is a method for increasing the volume of keratin fibers comprising the steps of:

(i) treating the keratin fibers by topical application of a cosmetic composition comprising, in a cosmetically acceptable medium:

(a) at least urea and/or an urea derivative selected from N,N-dimethylurea, N-(2-hydroxyethyl)urea, N-(3-hydroxypropyl)urea, N-(2-hydroxypropyl)urea, N-(2-hydroxy-2-methyl-prop-2-yl)urea, N-(1,3-dihydroxy-2-methyl-prop-2-yl)urea, N-(1,3-dihydroxy-2-hydroxymethyl-prop-2-yl)urea, and mixtures thereof; and
(b) at least one film-forming amino-silicone polymer selected from Polysilicone-35, Polysilicone-33, Polysilicone-29, and mixtures thereof; and

(ii) allowing the composition to remain in contact with the keratin fibers for a period ranging from about 5 seconds to about 96 hours.

This method increases the volume of keratin fibers, in particular human hair (expressed as hair diameter), without leaving the hair stiff or sticky, without leaving substantial adhesion between the hair fibers while maintaining the improvement in the volume of hair after washing it with a shampoo.

In an embodiment, in the method described herein, in step (ii) the composition is allowed to remain in contact with the keratin fibers for a period ranging from about 10 seconds to until the keratin fibers are dry.

In an embodiment, in the cosmetic composition for use in step (i) of the method described herein the component (a) is selected from urea, N,N-dimethylurea, N-(2-hydroxyethyl)urea and mixtures thereof.

In an embodiment, in the cosmetic composition for use in step (i) of the method described herein, the component

(a) comprises urea.

In an embodiment, in the cosmetic composition for use in step (i) of the method described herein, the component (b) comprises Polysilicone-29.

In an embodiment, in the cosmetic composition for use in step (i) of the method described herein, the total amount of the component (a) is in the range of about 0.5 to about 10 wt.%, based on the total weight of the composition.

In an embodiment, in the cosmetic composition for use in step (i) of the method described herein, the total amount of the component (b) is in the range of about 0.05 to about 10 wt.%, based on the total weight of the composition.

In an embodiment, the cosmetic composition for use in step (i) of the method described herein comprises, in a cosmetically acceptable medium:

(a) urea, N,N-dimethylurea, N-(2-hydroxyethyl)urea and mixtures thereof; and
(b) at least one film-forming amino-silicone polymer selected from Polysilicone-35, Polysilicone-33, Polysilicone-29, and mixtures thereof.

In an embodiment, the cosmetic composition for use in step (i) of the method described herein comprises, in a cosmetically acceptable medium, based on the total weight of the composition:

(a) about 0.5-10 wt.% of urea, N,N-dimethylurea, N-(2-hydroxyethyl)urea and mixtures thereof; and
(b) about 0.05-10 wt.% of at least one film-forming amino-silicone polymer selected from Polysilicone-35, Polysilicone-33, Polysilicone-29, and mixtures thereof.

In an embodiment, the cosmetic composition for use in step (i) of the method described herein comprises, in a cosmetically acceptable medium:

(a) urea; and
(b) Polysilicone-29.

In an embodiment, the cosmetic composition for use in step (i) of the method described herein comprises, in a cosmetically acceptable medium, based on the total weight of the composition:

(a) about 0.5-10 wt.% of urea; and
(b) about 0.05-10 wt.% of Polysilicone-29.

In an embodiment, in the method described herein, the keratin fibers are human hair.

In an embodiment, the cosmetic composition for use in step (i) of the method described herein, has a pH in the range from about 3.5 to about 8.5.

In an embodiment, in the method described herein, in step (ii) the keratin fibers are air-dried, towel-dried, blow-dried, heat-dried, steam-dried, radiation-dried or drying by combining two or more drying methods.

In an embodiment, the method described herein additionally comprises the step (iii) removing the cosmetic composition from the keratin fibers by rinsing with water or by washing the keratin fibers with a shampoo.

## DETAILED DESCRIPTION

Embodiments of the present disclosure are discussed in detail below. In describing embodiments, specific terminology is employed for the sake of clarity. However, the disclosure is not intended to be limited to the specific terminology so selected. While specific exemplary embodiments are discussed, it should be understood that this is done for illustration purposes only. A person skilled in the relevant art will recognize that other components and configurations can be used without parting from the spirit and scope of the disclosure. While a number of embodiments and features are described herein, it is to be understood that the various features of the disclosure and aspects of embodiments, even if described separately, may be combined unless mutually exclusive or contrary to the specific description. All references cited herein are incorporated by reference as if each had been individually incorporated.

The terms "about" or "approximately," as used herein, shall generally mean within 10 percent of a given value. Other than in the operating examples, or where otherwise indicated, all numbers expressing quantities of ingredients and/or reaction conditions are to be understood as being modified in all instances by the term "about".

The ranges defined in the specification include the end values as well, i.e. a range of 1 to 10 implies that both 1 and 10 are included in the range. For the avoidance of doubt, the applicant shall be entitled to any equivalents according to applicable law.

The term "keratin fibers," as used herein, refers to a family of proteins that occur in the vertebrates and exhibit a

protective function. In an embodiment, keratin fibers refer to human keratin fibers. In another embodiment, keratin fibers refer to human hair, such as human hair that grows on the scalp.

The singular form "a," "an" and "the," as used herein, include plural references unless the context clearly dictates otherwise. For example, the term "a peptide" includes a plurality of peptides, including mixtures thereof.

The term "comprising" refers to optional compatible components/steps that can be used provided that the important ingredients/steps are present.

The term "comprising" thus encompasses and includes the more restrictive terms "consisting of" and "consisting essentially of".

The term "cosmetic product" means any substance or mixture intended to be placed in contact with the external parts of the human body (epidermis, hair system, nails, lips and external genital organs) or with the teeth and the mucous membranes of the oral cavity with a view exclusively or mainly to cleaning them, perfuming them, changing their appearance, protecting them, keeping them in good condition or correcting body odors.

The term "cosmetic composition" (or cosmetic formulation) means any cosmetic product or preparation of the type described in Annex I ("Illustrative list by category of cosmetic products") of the Council Directive of the European Communities No. 76/768/EEC of July 27, 1976, known as the Cosmetics Directive, replaced by Regulation (EC) No 1223/2009 of the European Parliament and of the Council of 30 November 2009 on cosmetic products.

The cosmetic compositions can be formulated in a large number of types of product for the skin and/or the hair such as mousses, gels (in particular styling gels), masks for the face or the hair, conditioners, formulations for improving hairstyling, or for facilitating the combing or disentangling of the hair, for providing volume or sheen, rinsing formulations, compositions for dying or coloring the hair, hand and body lotions and oils, products for improving the moisturization of the skin, cleansing milks, make-up-removing compositions, creams or lotions for protecting against the sun and ultraviolet radiation, care and/or treatment milks and creams, anti-acne preparations, local analgesics, mascaras, products intended to be applied to the lips or other mucous membranes, sticks, deodorant and antiperspirant products, shaving lotions, bath oils, talc's and other compositions of the same type.

The term "hair product" means a cosmetic product which is intended to be applied on the hair of head or face, except eye lashes.

The term "film-forming," as used herein, refers to any compound that leaves a pliable, cohesive, and continuous covering over the hair when applied to the surface of the hair.

The term "amino-silicone," as used herein, refers to silicone functionalized with at least one amino group.

The term "polymer," as used herein, comprises copolymers (including terpolymers) and homopolymers.

The term "INCI" is the acronym of International Nomenclature Cosmetic Ingredient. INCI names are systematic names internationally recognized to identify cosmetic ingredients. They are developed by the International Nomenclature Committee (INC) and published by the Personal Care Products Council (PCPC) in the International Cosmetic Ingredient Dictionary and Handbook.

The term "CAS RN" is the acronym of CAS Registry Number, which is a unique identification number, assigned by the Chemical Abstracts Service (CAS) to every chemical substance described in the open scientific literature, in order to index the substance in the CAS Registry, which is a collection of disclosed chemical substance information.

The cosmetic composition for use in step (i) of the method described herein comprises, in a cosmetically acceptable medium:

(a) at least urea and/or an urea derivative selected from N,N-dimethylurea, N-(2-hydroxyethyl)urea, N-(3-hydroxypropyl)urea, N-(2-hydroxypropyl)urea, N-(2-hydroxy-2-methyl-prop-2-yl)urea, N-(1,3-dihydroxy-2-methyl-prop-2-yl)urea, N-(1,3-dihydroxy-2-hydroxymethyl-prop-2-yl)urea, and mixtures thereof; and

(b) at least one film-forming amino-silicone polymer selected from Polysilicone-35, Polysilicone-33, Polysilicone-29, and mixtures thereof.

Cosmetically acceptable medium

The cosmetically acceptable medium in the cosmetic composition for use in step (i) of the method described herein may be an aqueous medium including water and may include cosmetically acceptable organic solvents including alcohols such as ethyl alcohol, isopropyl alcohol, benzyl alcohol and phenylethyl alcohol, glycols or glycol ethers such as, for example, monomethyl, monoethyl and monobutyl ethers of ethylene glycol, propylene glycol or its ethers such as, for example, monomethyl ether of propylene glycol, butylene glycol, pentylene glycol, dipropylene glycol as well as the alkyl ethers of diethylene glycol such as, for example, monoethyl ether or monobutyl ether of diethylene glycol.

Water may be present in the range of about 30 to about 99 wt. %, or about 40 to about 95 wt. %, or about 45 to about 90 wt.%, based on the total weight of the composition. One or more organic solvents may be present in concentrations of between about 0.5 to about 20 wt. %, or about 2 to about 15 wt.%, based on the total weight of the cosmetic composition.

The urea and/or urea derivative

In the cosmetic composition for use in step (i) of the method described herein, component (a) may be selected from urea, N,N-dimethylurea, N-(2-hydroxyethyl)urea, N-(3-hydroxypropyl)urea, N-(2-hydroxypropyl)urea, N-(2-hydroxy-2-methyl-prop-2-yl)urea, N-(1,3-dihydroxy-2-methyl-prop-2-yl)urea, N-(1,3-dihydroxy-2-hydroxymethyl-prop-2-yl) urea, and mixtures thereof.

In one embodiment, in the cosmetic composition for use in step (i) of the method described herein, component (a) may be selected from urea (CAS RN 57-13-6), N,N-dimethylurea (CAS RN 96-31-1), N-(2-hydroxyethyl)urea (CAS RN 1320-51-0) and mixtures thereof.

In one embodiment, in the cosmetic composition for use in step (i) of the method described herein, component (a) comprises urea.

In one embodiment, in the cosmetic composition for use in step (i) of the method described herein, urea is the only component (a).

In the cosmetic composition for use in step (i) of the method described herein, the total amount of urea and/or urea derivative (a), may be in the range of about 0.5 to about 10 wt.%, or about 0.8 to about 7.5 wt.%, or about 1 to about 6 wt.%, or about 1.5 to about 5.5 wt.%, or about 2 to about 5 wt.%, based on the total weight of the cosmetic composition.

The film-forming amino-silicone polymer

In the cosmetic composition for use in step (i) of the method described herein, the film-forming amino-silicone polymer (b), may be selected from Polysilicone-35, Polysilicone-33, Polysilicone-29 and mixtures thereof.

According to the Personal Care Products Council (PCPC) On-Line INFOBASE, Polysilicone-35 is a siloxane polymer that conforms generally to the following formula:

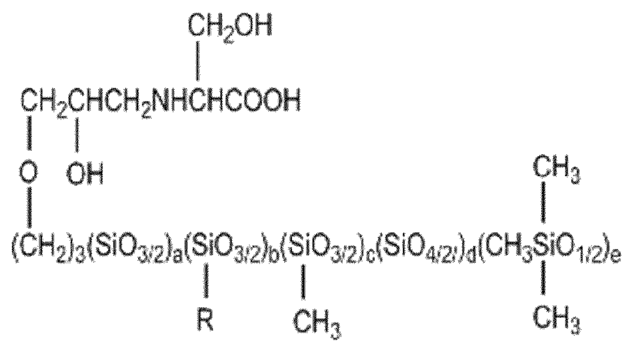

where R represents a $C_{6-10}$ alkyl chain.

According to the PCPC On-Line INFOBASE, Polysilicone-33 is a polysilsesquioxane that also contains dimethyl siloxane groups. It is produced by the hydrolysis and condensation of dimethyldimethoxysilane, Methyltrimethoxysilane, propyltrimethoxysilane, Aminopropyl Triethoxysilane, octyltriethoxysilane, and phenyltrimethoxysilane.

According to the PCPC On-Line INFOBASE, Polysilicone-29 is a complex silicone polymer formed by the reaction between a glycidoxypropyl-terminated dimethyl siloxane polymer, PEG-13 diglycidyl ether, diethylaminopropylamine, and aminopropyltriisopropoxysilane.

In one embodiment, in the cosmetic composition for use in step (i) of the method described herein, component (b), the film-forming amino-silicone polymer comprises Polysilicone-29.

A suitable film-forming amino-silicone polymer for use in the present disclosure is available under the trademark Silsoft(R) CLX-E by the company Momentive Performance Materials. Silsoft(R) CLX-E has as the INCI (International Nomenclature Cosmetic Ingredient) name "Dipropylene glycol and Polysilicone-29".

In the cosmetic composition for use in step (i) of the method described herein, the total amount of the film-forming amino-silicone polymer (b), may be in the range of about 0.05 to about 10 wt.%, or about 0.08 to about 5 wt.%, or about 0.1 to about 3 wt.%, or about 0.15 to about 2 wt.%, or about 0.15 to about 1.5 wt.% based on the total weight of the composition.

The cosmetic compositions

In another embodiment, the cosmetic composition for use in step (i) of the method described herein is a cosmetic composition for treating human hair.

The cosmetic composition for use in step (i) of the method described herein may comprise, in a cosmetically acceptable medium:

(a) at least urea and/or an urea derivative selected from N,N-dimethylurea, N-(2-hydroxyethyl)urea, N-(3-hydroxypropyl)urea, N-(2-hydroxypropyl)urea, N-(2-hydroxy-2-methyl-prop-2-yl)urea, N-(1,3-dihydroxy-2-methyl-prop-2-yl)urea, N-(1,3-dihydroxy-2-hydroxymethyl-prop-2-yl)urea, and mixtures thereof; and
(b) at least one film-forming amino-silicone polymer selected from Polysilicone-35, Polysilicone-33, Polysilicone-29, and mixtures thereof.

In the cosmetic composition for use in step (i) of the method described herein, the total amount of urea and/or urea derivative (a), may be in the range of about 0.5 to about 10 wt.%, or about 0.8 to about 7.5 wt.%, or about 1 to about 6 wt.%, or about 1.5 to about 5.5 wt.%, or about 2 to about 5 wt.%, based on the total weight of the cosmetic composition.

In the cosmetic composition for use in step (i) of the method described herein, the total amount of the film-forming amino-silicone polymer (b), may be in the range of about 0.05 to about 10 wt.%, or about 0.08 to about 5 wt.%, or about 0.1 to about 3 wt.%, or about 0.15 to about 2 wt.%, or about 0.15 to about 1.5 wt.% based on the total weight of the composition.

In an embodiment, the cosmetic composition for use in step (i) of the method described herein comprises, in a cosmetically acceptable medium, based on the total weight of the composition:

a) about 0.5-10 wt.% of at least urea and/or an urea derivative selected from N,N-dimethylurea, N-(2-hydroxyethyl)urea, N-(3-hydroxypropyl)urea, N-(2-hydroxypropyl)urea, N-(2-hydroxy-2-methyl-prop-2-yl)urea, N-(1,3-dihydroxy-2-methyl-prop-2-yl)urea, N-(1,3-dihydroxy-2-hydroxymethyl-prop-2-yl)urea, and mixtures thereof; and
b) about 0.05-10 wt.% of at least one film-forming amino-silicone polymer selected from Polysilicone-35, Polysilicone-33, Polysilicone-29, and mixtures thereof.

In an embodiment, the cosmetic composition for use in step (i) of the method described herein comprises, in a cosmetically acceptable medium, based on the total weight of the composition:

a) about 0.8-7.5 wt.% of at least urea and/or an urea derivative selected from N,N-dimethylurea, N-(2-hydroxyethyl)urea, N-(3-hydroxypropyl)urea, N-(2-hydroxypropyl)urea, N-(2-hydroxy-2-methyl-prop-2-yl)urea, N-(1,3-dihydroxy-2-methyl-prop-2-yl)urea, N-(1,3-dihydroxy-2-hydroxymethyl-prop-2-yl)urea, and mixtures thereof; and
b) about 0.08-5 wt.% of at least one film-forming amino-silicone polymer selected from Polysilicone-35, Polysilicone-33, Polysilicone-29, and mixtures thereof.

In an embodiment, the cosmetic composition for use in step (i) of the method described herein comprises, in a cosmetically acceptable medium, based on the total weight of the composition:

a) about 1-6 wt.% of at least urea and/or an urea derivative selected from N,N-dimethylurea, N-(2-hydroxyethyl)urea, N-(3-hydroxypropyl)urea, N-(2-hydroxypropyl)urea, N-(2-hydroxy-2-methyl-prop-2-yl)urea, N-(1,3-dihydroxy-2-methyl-prop-2-yl)urea, N-(1,3-dihydroxy-2-hydroxymethyl-prop-2-yl)urea, and mixtures thereof; and
b) about 0.1-3 wt.% of at least one film-forming amino-silicone polymer selected from Polysilicone-35, Polysilicone-33, Polysilicone-29, and mixtures thereof.

In an embodiment, the cosmetic composition for use in step (i) of the method described herein comprises, in a cosmetically acceptable medium, based on the total weight of the composition:

a) about 1.5-5.5 wt.% of at least urea and/or an urea derivative selected from N,N-dimethylurea, N-(2-hydroxyethyl)urea, N-(3-hydroxypropyl)urea, N-(2-hydroxypropyl)urea, N-(2-hydroxy-2-methyl-prop-2-yl)urea, N-(1,3-dihydroxy-2-methyl-prop-2-yl)urea, N-(1,3-dihydroxy-2-hydroxymethyl-prop-2-yl)urea, and mixtures thereof; and
b) about 0.15-2 wt.% of at least one film-forming amino-silicone polymer selected from Polysilicone-35, Polysilicone-33, Polysilicone-29, and mixtures thereof.

In an embodiment, the cosmetic composition for use in step (i) of the method described herein comprises, in a cosmetically acceptable medium, based on the total weight of the composition:

a) about 2-5 wt.% of at least urea and/or an urea derivative selected from N,N-dimethylurea, N-(2-hydroxyethyl)urea, N-(3-hydroxypropyl)urea, N-(2-hydroxypropyl)urea, N-(2-hydroxy-2-methyl-prop-2-yl)urea, N-(1,3-dihydroxy-2-methyl-prop-2-yl)urea, N-(1,3-dihydroxy-2-hydroxymethyl-prop-2-yl)urea, and mixtures thereof; and
b) about 0.15-1.5 wt.% of at least one film-forming amino-silicone polymer selected from Polysilicone-35, Polysilicone-33, Polysilicone-29, and mixtures thereof.

In an embodiment, the cosmetic composition for use in step (i) of the method described herein comprises, in a

cosmetically acceptable medium, based on the total weight of the composition:

(a) about 0.5-10 wt.% of urea, N,N-dimethylurea, N-(2-hydroxyethyl)urea and mixtures thereof; and
(b) about 0.05-10 wt.% of at least one film-forming amino-silicone polymer selected from Polysilicone-35, Polysilicone-33, Polysilicone-29, and mixtures thereof.

In an embodiment, the cosmetic composition for use in step (i) of the method described herein comprises, in a cosmetically acceptable medium, based on the total weight of the composition:

(a) about 0.8-7.5 wt.% of urea, N,N-dimethylurea, N-(2-hydroxyethyl)urea and mixtures thereof; and
(b) about 0.08-5 wt.% of at least one film-forming amino-silicone polymer selected from Polysilicone-35, Polysilicone-33, Polysilicone-29, and mixtures thereof.

In an embodiment, the cosmetic composition for use in step (i) of the method described herein comprises, in a cosmetically acceptable medium, based on the total weight of the composition:

(a) about 1-6 wt.% of urea, N,N-dimethylurea, N-(2-hydroxyethyl)urea and mixtures thereof; and
(b) about 0.1-3 wt.% of at least one film-forming amino-silicone polymer selected from Polysilicone-35, Polysilicone-33, Polysilicone-29, and mixtures thereof.

In an embodiment, the cosmetic composition for use in step (i) of the method described herein comprises, in a cosmetically acceptable medium, based on the total weight of the composition:

(a) about 1.5-5.5 wt.% of urea, N,N-dimethylurea, N-(2-hydroxyethyl)urea and mixtures thereof; and
(b) about 0.15-2 wt.% of at least one film-forming amino-silicone polymer selected from Polysilicone-35, Polysilicone-33, Polysilicone-29, and mixtures thereof.

In an embodiment, the cosmetic composition for use in step (i) of the method described herein comprises, in a cosmetically acceptable medium, based on the total weight of the composition:

(a) about 2-5 wt.% of urea, N,N-dimethylurea, N-(2-hydroxyethyl)urea and mixtures thereof; and
(b) about 0.15-1.5 wt.% of at least one film-forming amino-silicone polymer selected from Polysilicone-35, Polysilicone-33, Polysilicone-29, and mixtures thereof.

In an embodiment, the cosmetic composition for use in step (i) of the method described herein comprises, in a cosmetically acceptable medium, based on the total weight of the composition:

a) about 0.5-10 wt.% of urea; and
b) about 0.05-10 wt.% of Polysilicone-29.

In an embodiment, the cosmetic composition for use in step (i) of the method described herein comprises, in a cosmetically acceptable medium, based on the total weight of the composition:

a) about 0.8-7.5 wt.% of urea; and
b) about 0.08-5 wt.% of Polysilicone-29.

In an embodiment, the cosmetic composition for use in step (i) of the method described herein comprises, in a cosmetically acceptable medium, based on the total weight of the composition:

a) about 1-6 wt.% of urea; and
b) about 0.1-3 wt.% of Polysilicone-29.

In an embodiment, the cosmetic composition for use in step (i) of the method described herein comprises, in a cosmetically acceptable medium, based on the total weight of the composition:

a) about 1.5-5.5 wt.% of urea; and
b) about 0.15-2 wt.% of Polysilicone-29.

In an embodiment, the cosmetic composition for use in step (i) of the method described herein comprises, in a cosmetically acceptable medium, based on the total weight of the composition:

a) about 2-5 wt.% of urea; and
b) about 0.15-1.5 wt.% of Polysilicone-29.

In an embodiment, in the cosmetic composition for use in step (i) of the method described herein, the weight ratio between (a) urea and/or an urea derivative and (b) the film-forming amino-silicone polymer ranges from about 0.5:1 to about 50:1, or from about 1:1 to about 30:1, or from about 5:1 to about 20:1.

Other Components

The cosmetic composition for use in step (i) of the method described herein may contain a variety of other components to enhance the beneficial properties of this composition, as further described herein.

1. Thickening Agents

In an embodiment, the compositions of the present disclosure may contain one or more agents that will provide a thickening, or viscosity increasing effect, to the compositions. Suitable thickening agents include, but are not limited to, anionic, synthetic polymers, cationic, synthetic polymers, naturally occurring thickeners, such as nonionic guar gums, scleroglucan gums or xanthan gums, gum arabic, gum ghatti, karaya gum, tragacanth gum, carrageenan gum, agar-agar, locust bean gum, pectins, alginates, starch fractions, and derivatives such as amylose, amylopectin, and dextrins, as well as cellulose derivatives such as, for example, methylcellulose, carboxyalkylcelluloses, and hydroxyalkylcelluloses (such as hydroxyethylcellulose, hydroxyethyl ethylcellulose, hydroxypropylcellulose or hydroxypropyl methylcellulose), nonionic, fully synthetic polymers, such as polyvinyl alcohol or polyvinylpyrrolidinone; as well as inorganic thickeners, such as phyllosilicates, for example, bentonite, or smectites, such as montmorillonite or hectorite. Thickening agents may be included at from about 0.001-20 wt.%, or about 0.005-15 wt.%, or about 0.01-12 wt.%, based on the total weight of the composition.

2. Preservatives

"Preservatives", as used herein, are ingredients which prevent or retard microbial growth and thus help protect cosmetic products from spoilage. Preservatives may also protect the product from inadvertent contamination by the consumer during use. Suitable preservatives include, but are not limited to, methyl, ethyl, and propyl paraben, hydantoins, and the like. Preservatives may be included at about 0.0001-8 wt.%, or about 0.0005-7 wt.%, or about 0.001-5 wt.%, based on the total weight of the composition.

3. Chelating Agents

The compositions of the present disclosure may contain about 0.0001-5 wt.%, or 0.0005-3 wt.%, or 0.001-2 wt.%, based on the total weight of the composition, of one or more chelating agents. Chelating agents may be capable of complexing with and inactivating metallic ions in order to help prevent their adverse effects on the stability or effects of the composition. Suitable chelating agents include, but are not limited to, sodium citrate; nitrogen-containing polycarboxylic acids, such as Ethylenediaminetetraacetate (EDTA), and calcium, sodium, potassium or triethanolamine derivatives thereof, Hydroxyethyl Ethylenediamine Triacetic Acid (HEDTA), Ethylenediamine-N,N'-disuccinic acid (EDDS); and phosphonates, such as 1-hydroxyethane-1 ,1-diphosphonate (HEDP), and/or ethylenediamine tetramethylene phosphonate (EDTMP), and/or diethylenetriamine pentamethylene phosphonate (DTPMP), or sodium salts thereof.

4. pH Adjusters

In embodiments, acids, bases or buffering systems may be added to adjust the pH of the compositions of the present disclosure to the desired pH range. Suitable acids include hydrochloric acid, phosphoric acid, citric acid, and the like. Suitable bases include sodium or potassium hydroxide, monoethanolamine, diethanolamine, triethanolamine, aminomethyl propanol, and the like. pH adjusters may be included at from about 0.00001-8 wt.%, or about 0.00005-6 wt.%, or about 0 0001-5 wt.%, based on the total weight of the composition.

5. Pigments

The compositions of the present disclosure may contain inorganic pigments. Suitable inorganic pigments include, but are not limited to, those having a color index number as listed in the CTFA dictionary, 10th edition, 2004, hereby incorporated by reference.

6. Direct dyes

The compositions of the present disclosure may contain direct dyes. Direct dye, also called substantive dye, is a dye that adheres to its substrate by non-ionic forces, e.g., without help from other chemicals. For purposes of this disclosure dyes include direct dyes selected from anionic, cationic and non-ionic nitro dyes.

Examples of anionic direct dyes include, but are not limited to, Bromophenol Blue, Tetrabromophenol Blue, Acid Black 1, Acid Blue 1, Acid Blue 3, Food Blue 5, Acid Blue 7, Acid Blue 9, Acid Blue 74, Acid Orange 3, Acid Orange 6, Acid Orange 7, Acid Orange 10, Acid Red 1, Acid Red 14, Acid Red 18, Acid Red 27, Acid Red 50, Acid Red 52, Acid Red 73, Acid Red 87, Acid Red 88, Acid Red 92, Acid Red 155, Acid Red 180, Acid Violet 9, Acid Violet 43, Acid Violet 49, Acid Yellow 1, Acid Yellow 23, Acid Yellow 3, Food Yellow No. 8, D&C Brown No. 1, D&C Green No. 5, D&C Green No. 8, D&C Orange No. 4, D&C Orange No. 10, D&C Orange No. 11, D&C Red No. 21, D&C Red No. 27, D&C Red No. 33, D&C Violet 2, D&C Yellow No. 7, D&C Yellow No. 8, D&C Yellow No. 10, FD&C Red 2, FD&C Red 40, FD&C Red No. 4, FD&C Yellow No. 5, FD&C Yellow No. 6, FD&C Blue 1, Food Black 1, Food Black 2, Disperse Black 9 and Disperse Violet 1, and their alkali metal salts such as sodium or potassium.

Examples of cationic dyes include, but are not limited to, Basic Blue 6, Basic Blue 7, Basic Blue 9, Basic Blue 26, Basic Blue 41, Basic Blue 99, Basic Brown 4, Basic Brown 16, Basic Brown 17, Natural Brown 7, Basic Green 1, Basic Red 2, Basic Red 12, Basic Red 22, Basic Red 76, Basic Violet 1, Basic Violet 2, Basic Violet 3, Basic Violet 10, Basic Violet 14, Basic Yellow 57, Basic red 51, Basic Yellow 87 and Basic Orange 31.

Examples of non-ionic nitro dyes (HC dyes) include, but are not limited to, HC Blue No. 2, HC Blue No. 4, HC Blue No. 5, HC Blue No. 6, HC Blue No. 7, HC Blue No. 8, HC Blue No. 9, HC Blue No. 10, HC Blue No. 11, HC Blue No. 12, HC Blue No. 13, HC Blue No. 15, Blue No. 16, HC Blue No. 18, HC Brown No. 1, HC Brown No. 2, HC Green No. 1, HC Orange No. 1, HC Orange No. 2, HC Orange No. 3, HC Orange No. 5, HC Red BN, HC Red No. 1, HC Red No. 3, HC Red No. 7, HC Red No. 8, HC Red No. 9, HC Red No. 10, HC Red No. 11, HC Red No. 13, HC Red No. 14, HC Red No. 18, HC Red No. 54, HC Violet BS, HC Violet No. 1, HC Violet No. 2, HC Yellow No. 2, HC Yellow No. 4, HC Yellow No. 5, HC Yellow No. 6, HC Yellow No. 7, HC Yellow No. 8, HC Yellow No. 9, HC Yellow No. 10, HC Yellow No. 11, HC Yellow No. 12, HC Yellow No. 13, HC Yellow No. 14, HC Yellow No. 15, 2-Amino-6-chloro-4-nitrophenol, picramic acid, 1,2-Diamino-4-nitrobenzol, 1,4-Diamino-2-nitrobenzol, 3-Nitro-4-aminophenol, 1-Hydroxy-2-amino-3-nitrobenzol and 2-hydroxyethylpicramic acid.

7. Fragrance or perfumes

The compositions of the present disclosure may include one or more fragrances or perfumes. For purposes of this disclosure, the term "fragrance" refers to any substance, natural or synthetic, used to impart an odor to a product.

For purposes of this disclosure, the term "perfume" refers to any mixture of fragrant essential oils and aroma compounds, fixatives, and solvents used to give the human body, objects, and living spaces a lasting and pleasant smell.

8. Anionic surfactants

The compositions of the present disclosure may include one or more anionic surfactants. Non-limiting examples of anionic surfactants include, but are not limited to, alkyl ether carboxylates, alkyl sulfates, alkyl ether sulfates, amide ether sulfates, alkyl glyceride sulfates, olefin sulfonates, alkyl-aryl sulfonates, sulfosuccinates, sulfo fatty acid esters, fatty acid isethionates, fatty acid taurides, phosphate esters, acyl glutamates, acyl peptides, acyl sarcosides, and mixtures thereof.

Examples of alkyl ether carboxylates, alkyl sulfates, alkyl ether sulfates, olefin sulfonates, and mixtures thereof include Potassium Laureth-4 Carboxylate, Sodium Laureth-4 Carboxylate, Sodium Laureth-5 Carboxylate, Sodium Laureth-6 Carboxylate, and Sodium Laureth-11 Carboxylate, Ammonium Laureth Sulfate, Monoethanolamine (MEA)-Laureth Sulfate, Monoisopropanolamine (MIPA)-Laureth Sulfate, Sodium Laureth Sulfate, and Triethanolamine (TEA)-Laureth Sulfate, Sodium C14-C16 Olefin Sulfonate, and mixtures thereof.

9. Cationic surfactants

The compositions of the present disclosure may include one or more cationic surfactants. Non-limiting examples of cationic surfactants include, but are not limited to, alkylimidazolines, tetra alkyl(-aryl) quaternary ammonium salts

(quats), heterocyclic ammonium salts, quaternized alkyl polyglycosides, quaternized derivatives of polyalkanolamine esters (esterquats), and mixtures thereof.

Examples of C6-C24 alkyl trimethyl quaternary ammonium salts, C6-C24 alkyl dimethyl benzyl quaternary ammonium salts, C6-C24 dialkyl dimethyl quaternary ammonium salts, and mixtures thereof include Laurtrimonium bromide, Laurtrimonium chloride, Myrtrimonium bromide, Myrtrimonium chloride, Cetrimonium bromide, Cetrimonium chloride, Cetrimonium methosulfate, Steartrimonium bromide, Steartrimonium chloride, Steartrimonium methosulfate, Behentrimonium bromide, Behentrimonium chloride, Behentrimonium methosulfate, Ceteartrimonium chloride, Cocotrimonium chloride, Cocotrimonium methosulfate, Soytrimonium chloride, Octyl dodecyl trimethyl ammonium chloride, Dodecyl hexadecyl trimethyl ammonium bromide, Dodecyl hexadecyl trimethyl ammonium chloride, Benzalkonium chloride, benzyl-C10-16-alkyldimethylammonium chloride, benzyl-C12-14-alkyldimethylammonium chloride, Didecyldimonium chloride, Dilauryldimonium chloride, Distearyldimonium chloride, Behenoyl PG-Trimonium Chloride, Dioleoylethyl Hydroxyethylmonium Methosulfate, and mixtures thereof.

10. Nonionic surfactants

The compositions of the present disclosure may include one or more nonionic surfactants. Non-limiting examples of nonionic surfactants include, but are not limited to, polyethoxylated and/or polypropoxylated C6-C24 fatty alcohols, polyethoxylated and/or polypropoxylated fatty acids, alkylphenols, alpha-diols or alcohols having a fatty chain containing, for example, 8 to 18 carbon atoms, ethylene oxide or propylene oxide groups to range from 2 to 50; copolymers of ethylene oxide and of propylene oxide, polyethoxylated fatty amides having from 2 to 30 mol of ethylene oxide; polyglycerolated fatty amides containing on average 1 to 5, or 1.5 to 4, glycerol groups; polyethoxylated fatty amines having 2 to 30 mol of ethylene oxide; oxyethylenated fatty acid esters of sorbitan having from 2 to 30 mol of ethylene oxide; polyethoxylated castor oil, polyethoxylated hydrogenated castor oil, fatty acid esters of sucrose; fatty acid esters of polyethylene glycol; alkylpolyglycosides; N-alkylglucamine derivatives; amine oxides such as (C10-C14) alkylamine oxides or N-acylaminopropylmorpholine oxides.

11. Amphoteric or zwitterionic surfactants

The compositions of the present disclosure may include one or more amphoteric or zwitterionic surfactants. Non-limiting examples of amphoteric or zwitterionic surfactants include, but are not limited to, aliphatic secondary or tertiary amine derivatives in which the aliphatic radical is a linear or branched chain containing 8 to 18 carbon atoms and containing at least one water-soluble anionic group (for example carboxylate, sulphonate, sulphate, phosphate or phosphonate); (C8-C20) alkylbetaines, sulphobetaines, (C8-C20) alkylamido (C1-C6) alkylbetaines or (C8-C20) alkylamido (C1-C6) alkylsulphobetaines; amine derivatives, disodium cocoamphodiacetate, disodium lauroamphodiacetate, disodium caprylamphodiacetate, disodium capryloamphodiacetate, disodium cocoamphodipropionate, disodium lauroamphodipropionate, disodium caprylamphodipropionate, disodium capryloamphodipropionate, lauroamphodipropionic acid, and cocoamphodipropionic acid.

12. Petroleum hydrocarbons (mineral oils, paraffins and waxes)

The compositions of the present disclosure may include one or more petroleum hydrocarbons. For purposes of this disclosure petroleum hydrocarbons refer to mineral oils, paraffins and waxes based on petroleum. Examples include, but are not limited to, hard paraffin, liquid paraffin (Liquid Petrolatum or Paraffinum Liquidum), light liquid paraffin (Light Liquid Petrolatum or Paraffinum Perliquidium), white soft paraffin (White Petrolatum), yellow soft paraffin (Yellow Petrolatum), macrocrystalline paraffin waxes (which are mixtures that consist mainly of saturated C18-C30 hydrocarbons and smaller amounts of iso-alkanes and cycloalkanes with a molecular weight between 250 and 450 g/mol and, although they are solids at room temperature, they have low melting points, usually between 40°C and 60°C), microcrystalline paraffin waxes (which consist of C40-C55 compounds that contain, in addition to normal hydrocarbons, included in the group are iso-alkanes and naphtenes with long alkyl side-chains, the iso-alkanes forming microcrystals, the microcrystalline paraffin waxes having mean molecular weights between 500 and 800 g/mol, being solids at room temperature, and having melting points between 60°C and 90°C), or mixtures thereof.

13. C6-C24 fatty alcohols

The compositions of the present disclosure may include one or more C6-C24 fatty alcohols. C6-C24 fatty alcohols from vegetable fats and oils include cotton, safflower, coconut, rapeseed, linseed, palm, palm kernel, sunflower, olein, olive, olive pomace, castor oil, soy, tall oil, etc., totally or partially hydrogenated, as well as purified or synthetic fatty alcohols such as caproyl alcohol, capryl alcohol, capric alcohol, lauryl alcohol, myristyl alcohol, palmytil (cetyl) alcohol,

palmitoyl alcohol, stearyl alcohol, isostearyl alcohol, 2-octyldodecanol, 2-ethylhexanoyl alcohol, oleyl alcohol, ricinoleyl alcohol, elaidyl alcohol, petroselinic alcohol, linoleyl alcohol, linolenyl alcohol, arachidyl alcohol, gadoleyl alcohol, behenyl alcohol and erucyl alcohol, or technical grade mixtures.

14. Vegetable fats and oils

The compositions of the present disclosure may include one or more vegetable fats and/or oils. For purposes of this disclosure, vegetable fats and oils are linear and/or branched esters, linear or branched, saturated and/or unsaturated alkanecarboxylic acids with a chain length of 1 up to 30 carbon atoms, saturated and/or unsaturated alcohols with a chain length of 1 up to 30 carbon atoms; or linear and/or branched esters of aromatic carboxylic acids, or saturated and/or unsaturated alcohols with a chain length of 1 up to 30 carbon atoms. These oils can be selected from, for example, isopropyl myristate, isopropyl palmitate, isopropyl stearate, isopropyl oleate, n-butyl stearate, n-hexyl laurate, n-decyl oleate, isooctyl stearate, isononyl stearate, isononyl isononanoate, 2-ethylhexyl laurate, 2-ethylhexyl palmitate, 2-ethylhexyl cocoate, 2-hexyldecyl stearate, 2- ethylhexyl isostearate, 2-octyldodecyl palmitate, cetyl palmitate, stearyl palmitate, oleyl palmitate, oleyl oleate, oleyl erucate, erucyl oleate, erucyl erucate, as well as synthetic, semisynthetic and natural mixtures esters, such as jojoba oil (a natural mixture of esters of monounsaturated monocarboxylic acids with a C18-C24 chain with also monounsaturated monoalcohols and with a long C18-C24 chain).

Other examples of vegetable fats and oils include ester oils such as sugar esters or diesters of C12-C24 fatty acids. The term "sugar" means compounds comprising several alcohol functional groups, with or without an aldehyde or ketone function, and which comprise at least 4 carbon atoms. These sugars may be monosaccharides, oligosaccharides or polysaccharides.

Non-limiting examples of sugars that may be used according to the present disclosure include sucrose (or saccharose), glucose, galactose, ribose, fucose, maltose, fructose, mannose, arabinose, xylose and lactose, and derivatives thereof, including for example, alkyl derivatives such as methyl derivatives, for instance methylglucose. Non-limiting examples of the sugar esters of fatty acids that may be used according to this disclosure include those from the group comprising esters or mixtures of esters of sugars described above and of linear or branched, saturated or unsaturated C12-C24 fatty acids.

The esters may be chosen from mono-, di-, tri-, tetraesters and polyesters, and mixtures thereof. These esters may be chosen from, for example, oleates, laurates, palmitates, myristates, behenates, cocoates, stearates, linoleates, linolenates, caprates and arachidonates, or mixtures thereof such as oleo-palmitate, oleo-stearate and palmito-stearate mixed esters. The sucrose, glucose or methylglucose monoesters and diesters and for example sucrose, glucose or methylglucose mono- or dioleates, stearates, behenates, oleopalmitates, linoleates, linolenates and oleostearates, constitute sugar esters or diesters of C12-C24 fatty acids that are suitable in the context of the present disclosure. One example is methylglucose dioleate.

Other suitable oils of the type of esters of saturated alkane carboxylic acids and alcohols are fatty acid methyl esters, such as C12-C24 fatty acid methyl esters from animal and vegetable fats and oils such as cotton, safflower, coconut, rapeseed, linseed, palm, palm kernel, sunflower, olein, olive, olive pomace, castor oil, soy, tall oil, etc., totally or partially hydrogenated, as well as purified or synthetic fatty acids such as caproic acid, caprylic acid, capric acid, lauric acid, myristic acid, palmitic acid (cetylic acid), palmitoleic acid, stearic acid, isostearic acid, 2-ethylhexanoic acid, oleic acid, ricinoleic acid, elaidic acid, petroselinic acid, linoleic acid, linolenic acid, arachidic acid, gadoleic acid, behenic acid and erucic acid, or mixtures thereof.

Other suitable vegetable fats and oils according to the present disclosure are fatty acid triglycerides, including for example, linear and/or branched triglycerin esters, saturated and/or unsaturated alkanecarboxylic acids with a chain length of 6 up to 24 carbon atoms, such as 10 up to 18 carbon atoms. The fatty acids esterifying the different positions of glycerin can be different, giving rise to a large amount of possible combinations, including positional combinations. The position of the different fatty acids in natural triglycerides is not random, but rather it depends on the origin of the fat. The most simple triglycerides are those constituted by a sole fatty acid.

Fatty acid triglycerides can be chosen, for example, from synthetic, semi-synthetic and natural oils, for example avocado oil, almond oil, hazelnut oil, babassu palm oil, borage oil, peanut oil, canola oil, hemp oil, milk thistle oil, safflower oil, chufa oil, coconut oil, rapeseed oil, black cumin oil, wheat germ oil, sunflower oil, linseed oil, macadamia nut oil, corn oil, walnut oil, olive oil and its by-products such as olive pomace oil, palm oil and its fractions such as palm olein and palm stearin, evening primrose oil, rosehip oil, castor oil, rice bran oil, apricot kernel oil, cottonseed oil, pumpkinseed oil, palm kernel oil and its fractions such as palm kernel olein and palm kernel stearin, grape seed oil, sesame oil, soy oil, cocoa butter, shea butter and the like.

15. Natural waxes

The compositions of the present disclosure may include one or more natural waxes. Natural waxes according to

the present disclosure, include but are not limited to, candelilla wax, carnauba wax, Japan wax, esparto wax, cork wax, guaruma wax, rice wax, sugar cane wax, ouricury wax, montan wax, beeswax, shellac wax, espermaceti, wool lanolin (wax), uropygial fat wax, ceresin waxes, peat waxes, ozokerite, as well as chemically modified waxes (hard waxes) for example, montan wax esters, waxes obtained by the Fischer-Tropsch process, hydrogenated jojoba waxes and synthetic waxes.

16. Silicones different from the film-forming amino-silicone polymer

The compositions of the present disclosure may include one or more silicones other than the film-forming amino-silicone polymer or the silane derivative of a hydrolyzed protein or peptide or amino acid. Non-limiting examples of silicones different from the film-forming amino-silicone polymer or the silane derivative of a hydrolyzed protein or peptide or amino acid in the context of the present disclosure include, but are not limited to, cyclic and/or linear silicones, which can be found as monomers generally characterized by structural elements such as:

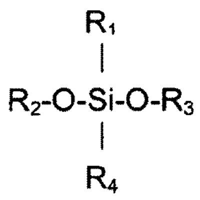

where the silicon atoms can be substituted by alkyl or aryl radicals the same or different, represented here generally by $R_1$-$R_4$ groups.

Linear silicones with siloxane units suitable according to the present disclosure are generally characterized by structural elements such as:

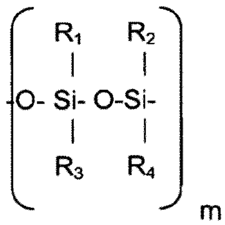

where the silicon atoms can be substituted by alkyl or aryl radicals the same or different, represented here in general by $R_1$-$R_4$ groups (meaning the number of different radicals is not limited to 4), and m can range from 2 to 200.000.

Cyclic silicones suitable according to the present disclosure are generally characterized by structural elements such as:

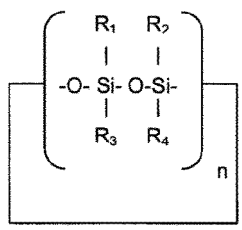

where the silicon atoms can be substituted by alkyl or aryl radicals the same or different, represented here generally by $R_1$-$R_4$ groups (meaning the number of different radicals is not limited to 4), and n can range from 3/2 to 20. Fractional values of n indicate that odd numbers of siloxane groups may be present in the ring.

Examples include a cyclic methyl siloxane having the formula $[(CH_3)_2SiO]x$ in which x is 3-6, or short chain linear methyl siloxanes having the formula $((CH_3)_2SiO[(CH_3)_2SiO]_ySi(CH_3)_3$ in which y is 0-5.

Some suitable cyclic methyl siloxanes are hexamethylcyclotrisiloxanes (D3), octamethylcyclotetrasiloxane (D4), decamethylcyclopentasiloxane (D5) (cyclomethicone) and dodecamethylcyclohexasiloxane (D6).

Some suitable short linear methyl siloxane are hexamethyldisiloxane (MM), octamethyltrisiloxane (MDM), decamethyltetrasiloxane (MD2M), dodecamethylpentasiloxane (MD3M), tetradecamethylhexasiloxane (MD4M), and hexadecamethylheptasiloxane (MD5M).

Long chain linear siloxanes such as phenyltrimethicone, bis(phenylpropyl)dimethicone, dimethicone, dimethiconol, cetyldimethicone and behenoxy dimethicone are also suitable silicones according to the present disclosure.

17. Cationic Polymers

The compositions of the present disclosure may include one or more cationic polymers. The term "cationic polymer" as used herein refers to a macromolecule (polymer) that contains at least one monomer bearing a quaternary ammonium group.

Examples of suitable cationic polymers include, but are not limited to, those polymers known by their International Nomenclature for Cosmetic Ingredients (INCI) name as Polyquaternium. Some examples of those are Polyquaternium-1, Polyquaternium-2, Polyquaternium-4, Polyquaternium-5, Polyquaternium-6, Polyquaternium-7, Polyquaternium-8, Polyquaternium-9, Polyquaternium-10, Polyquaternium-11, Polyquaternium-12, Polyquaternium-13, Polyquaternium-14, Polyquaternium-15, Polyquaternium-16, Polyquaternium-17, Polyquaternium-18, Polyquaternium-19, Polyquaternium-20, Polyquaternium-22, Polyquaternium-24, Polyquaternium-27, Polyquaternium-28, Polyquaternium-29, Polyquaternium-30, Polyquaternium-31, Polyquaternium-32, Polyquaternium-33, Polyquaternium-34, Polyquaternium-35, Polyquaternium-36, Polyquaternium-37, Polyquaternium-39, Polyquaternium-42, Polyquaternium-43, Polyquaternium-44, Polyquaternium-45, Polyquaternium-46, Polyquaternium-47, Polyquaternium-48, Polyquaternium-49, Polyquaternium-50, Polyquaternium-51, Polyquaternium-52, Polyquaternium-53, Polyquaternium-54, Polyquaternium-55, Polyquaternium-56, Polyquaternium-57, Polyquaternium-58, Polyquaternium-59, Polyquaternium-60, Polyquaternium-61, Polyquaternium-62, Polyquaternium-63, Polyquaternium-64, Polyquaternium-65, Polyquaternium-66, Polyquaternium-67, Polyquaternium-68, Polyquaternium-69, Polyquaternium-70, Polyquaternium-71, Polyquaternium-72, Polyquaternium-73, Polyquaternium-74, Polyquaternium-75, Polyquaternium-76, Polyquaternium-77, Polyquaternium-78, Polyquaternium-79, Polyquaternium-80, Polyquaternium-81, Polyquaternium-82, Polyquaternium-83, Polyquaternium-84, Polyquaternium-85, Polyquaternium-86, Polyquaternium-87, Polyquaternium-88, Polyquaternium-91, Polyquaternium-92, Polyquaternium-94, Polyquaternium-95, Polyquaternium-96, Polyquaternium-98, Polyquaternium-99, Polyquaternium-100, Polyquaternium-102, Polyquaternium-104, Polyquaternium-109, Polyquaternium-110, Polyquaternium-111, Polyquaternium-112, Polyquaternium-113 and Polyquaternium-114. Other suitable cationic polymers include those known by their International Nomenclature for Cosmetic Ingredients (INCI) name as Guar hydroxypropyl trimonium chloride, Polyacrylamidopropyltrimonium Chloride, Polymethacrylamidopropyltrimonium Chloride and Polymethacrylamidopropyltrimonium Methosulfate.

18. Non-ionic polymers

The compositions of the present disclosure may include one or more non-ionic polymers. Non-limiting examples of non-ionic polymers include, but are not limited to: nonionic guar gums and modified nonionic guar gums, which may be those modified with C1-C6 hydroxyalkyl groups; biopolysaccharide gums of microbial origin such as scleroglucan gum or xanthan gum; gums derived from plant exudates, such as gum arabic, ghatti gum, karaya gum, tragacanth gum, carrageenan, agar and carob gum; pectins; alginates; starches; hydroxy (C1-C6) alkylcelluloses and carboxy (C1-C6) alkylcelluloses; celluloses modified with groups comprising at least one fatty chain; non-limiting examples include hydroxyethylcelluloses modified with groups comprising at least one fatty chain, such as alkyl, arylalkyl or alkylaryl groups, or mixtures thereof, and wherein the alkyl groups are, for example, C8-C22; or those modified with polyalkylene glycol alkylphenyl ether groups; hydroxypropyl guars modified with groups comprising at least one C8-C22 fatty chain, copolymers of vinylpyrrolidone and of hydrophobic monomers comprising a fatty chain, such as vinylpyrrolidone/hexadecene copolymer or vinylpyrrolidone/eicosene copolymer; copolymers of C1-C6 alkyl acrylates or methacrylates and of amphiphilic monomers comprising at least one fatty chain; copolymers of hydrophilic acrylates or methacrylates and of hydrophobic monomers comprising at least one fatty chain, such as the polyethylene glycol methacrylate/lauryl methacrylate copolymer; polymers with an aminoplast ether skeleton comprising at least one fatty chain; and polyurethane polyethers comprising in their chain both hydrophilic blocks, for example of polyoxyethylenated nature, and hydrophobic blocks that may be aliphatic blocks alone and/or cycloaliphatic and/or aromatic blocks.

19. Amphoteric polymers

The compositions of the present disclosure may include one or more amphoteric polymers. Non-limiting examples of amphoteric polymers include, but are not limited to, amphoteric polysaccharides such as Carboxymethylchitosan or N-[(2'-Hydroxy-2',3'-dicarboxy)ethyl]chitosan; Amphoteric Urethanes; Modified Potato Starch; Methacryloyl Ethyl Betaine/Acrylates Copolymer; Acrylates/Lauryl Acrylate/Stearyl Acrylate/Ethylamine Oxide Methacrylate; and Acrylic acid/(meth)acrylamidopropyl-trimethylamonium chloride/stearyl methacrylate terpolymer.

20. Anionic polymers

The compositions of the present disclosure may include one or more anionic polymers. Non-limiting examples of anionic polymers include, but are not limited to, those polymers known by their International Nomenclature for Cosmetic Ingredients (INCI) name as Shellac, Acrylates Copolymer, Acrylates/C1-2 Succinates/Hydroxyacrylates Copolymer, Acrylic Esters (and) Methacrylic Esters Copolymer, AMP-Acrylates/Allyl Methacrylate Copolymer, Diglycol/Cyclohexanedimethanol/Isophtalates/Sulfoisophtalates Copolymer, Diglycol/Isophtalates/Sulfoisophtalates Copolymer, Isobutylene Ethylmaleimide/Hydroxyethylmaleimide Copolymer, Glycerin and Diglycol/Cyclohexanedimethanol/Isophtalates/-Sulfoisophtalates Copolymer, Methacrylate Acid/Sodium Acrylamidomethyl Propane Sulfonate Copolymer, Octylacrylamide/Acrylates/Butylaminoethylmethacrylate Copolymer, Polyurethane (and) Acrylates Copolymer, PVM/MA Copolymer, PVP/Ethyl Methacrylate/Methacrylic Acid Terpolymer, PVP/Polycarbamyl Polyglycol Ester, VA/Crotonates Copolymer, VA/Crotonates/Vinyl Neodecanoate Copolymer, VP/Acrylates/Lauryl Methacrylate Copolymer, and their cosmetically acceptable salts.

21. Other additives

The compositions of the present disclosure may include one or more additives other than those described above. Non-limiting examples of additives different from those listed above include, but are not limited to, Vitamins and Provitamins, such as beta-carotene, retinal, retinol, retinoic acid, biotin, panthenol, panthenyl ethyl ether, ascorbic acid, ascorbyl glucoside, ascorbyl palmitate, ascorbyl phosphate, tocopherol, tocopheryl acetate, tocopheryl glucoside, tocopheryl palmitate, and the like; hair strengthening agents such as maleic acid, hydroxypropylgluconamide (and) hydroxypropylammonium gluconate, and the like; antioxidants (ingredients employed in cosmetics to prevent or retard product spoilage from rancidity or deterioration from reaction with oxygen); moisturizers such as allantoin; natural extracts (substances or active ingredients with desirable properties that are removed from a plant, flower, alga, fungus or bacteria); UV filters (ingredients used to absorb or reflect the UV rays that are contained in sun light or in artificial light); or mixtures thereof.

In an embodiment, the cosmetic composition for use in step (i) of the method described herein comprises, in a cosmetically acceptable medium,

(a) at least urea and/or an urea derivative selected from N,N-dimethylurea, N-(2-hydroxyethyl)urea, N-(3-hydroxypropyl)urea, N-(2-hydroxypropyl)urea, N-(2-hydroxy-2-methyl-prop-2-yl)urea, N-(1,3-dihydroxy-2-methyl-prop-2-yl)urea, N-(1,3-dihydroxy-2-hydroxymethyl-prop-2-yl)urea, and mixtures thereof;
(b) at least one film-forming amino-silicone polymer selected from Polysilicone-35, Polysilicone-33, Polysilicone-29, and mixtures thereof; and
(c) optionally, at least one additional cosmetic ingredient selected from thickening agents; preservatives; chelating agents; pH adjusters; pigments; direct dyes; fragrance or perfumes; anionic surfactants; cationic surfactants; nonionic surfactants; amphoteric or zwitterionic surfactants; petroleum hydrocarbons (mineral oils, paraffins and waxes); C6-C24 fatty alcohols; vegetable fats and oils; natural waxes; silicones different from the film-forming amino-silicone polymer or the silane derivative of a hydrolyzed protein or peptide or amino acid; cationic polymers; non-ionic polymers; anionic polymers; and other additives such as vitamins and provitamins; hair strengthening agents; antioxidants; moisturizers; natural extracts; UV filters; or mixtures thereof.

In an embodiment, the cosmetic composition for use in step (i) of the method described herein comprises, in a cosmetically acceptable medium, based on the total weight of the composition:

a) about 0.5-10 wt.% of at least urea and/or an urea derivative selected from N,N-dimethylurea, N-(2-hydroxyethyl)urea, N-(3-hydroxypropyl)urea, N-(2-hydroxypropyl)urea, N-(2-hydroxy-2-methyl-prop-2-yl)urea, N-(1,3-dihydroxy-2-methyl-prop-2-yl)urea, N-(1,3-dihydroxy-2-hydroxymethyl-prop-2-yl)urea, and mixtures thereof;
b) about 0.05-10 wt.% of at least one film-forming amino-silicone polymer selected from Polysilicone-35, Polysilicone-33, Polysilicone-29, and mixtures thereof; and
c) optionally, at least one additional cosmetic ingredient selected from thickening agents; preservatives; chelating agents; pH adjusters; pigments; direct dyes; fragrance or perfumes; anionic surfactants; cationic surfactants; nonionic surfactants; amphoteric or zwitterionic surfactants; petroleum hydrocarbons (mineral oils, paraffins and waxes); C6-C24 fatty alcohols; vegetable fats and oils; natural waxes; silicones different from the film-forming amino-silicone polymer or the silane derivative of a hydrolyzed protein or peptide or amino acid; cationic polymers; non-ionic polymers; anionic polymers; and other additives such as vitamins and provitamins; hair strengthening agents; antioxidants; moisturizers; natural extracts; UV filters; or mixtures thereof.

Suitable cosmetic forms

No particular limitation is imposed on the form of the cosmetic composition for use in step (i) of the method described herein. Examples include hair sprays, setting lotions, mousses, gels, waxes, creams, pomades, gums or serums (Thomas Clausen et al., Hair Preparations, Ullmann's Encyclopedia of Industrial Chemistry, 2006).

In embodiments, the compositions are formulated as a hair spray, a setting lotion or a mousse.

Hair sprays may be aerosol sprays, which may be two-phase aerosols in which both a liquid and a gaseous phase are present inside the container; or pump sprays (non-aerosol sprays), which do not employ a propellant gas and the energy needed to swirl and atomize the liquid must be supplied by a pump, which is operated with a finger or through a lever.

Setting lotions may be thin, aqueous-alcohol solutions in single- or multiple-application packages.

Mousses may be thin, aqueous or aqueous-alcohol solutions in, for example, alumina cans with a propellant; or non-aerosol mousses, where the mousse is generated through use of a squeeze or pump dispenser with a riddle in the head of the pump. The liquid is mixed with air in the head of the pump and dispensed as mousse.

The cosmetic composition for use in step (i) of the method described herein may have a viscosity in the range of about 1 mPas to about 250,000 mPas, or in the range of about 5 mPas to about 200,000 mPas, or in the range of about 10 mPas to about 50,000 mPas, or in the range of about 100 mPas to about 25,000 mPas, or in the range of about 1,000 to about 15,000 mPas, measured with a Brookfield Dial Reading Viscometer LVT (spindle F at 0.3 rpm) at 25°C under atmospheric conditions.

The cosmetic composition for use in step (i) of the method described herein may have a pH in the range from about 3.5 to about 8.5, or about 4.0 to about 8.0.

In one embodiment, the cosmetic compositions for use in step (i) of the method described are "leave-on" compositions. The term "leave-on" as used herein refers to compositions that contain ingredients that are intended to be deposited and left on the hair for extended periods (i.e. several hours or days) until the ingredients are subsequently removed by water and/or shampooing the hair.

In one embodiment, the cosmetic compositions for use in step (i) of the method described are "rinse-off" compositions. The term "rinse-off" as used herein refers to compositions that contain ingredients intended to be applied and left on the hair for short periods of time (i.e. about 5 s to about 50 min, or about 10 s to 45 min) and subsequently be removed by water and/or shampooing the hair.

Treatment process

In an embodiment, it is described a method for increasing the volume of keratin fibers comprising the steps of:

(i) treating the keratin fibers by topical application of a cosmetic composition comprising, in a cosmetically acceptable medium:

(a) at least urea and/or an urea derivative selected from N,N-dimethylurea, N-(2-hydroxyethyl)urea, N-(3-hydroxypropyl)urea, N-(2-hydroxypropyl)urea, N-(2-hydroxy-2-methyl-prop-2-yl)urea, N-(1,3-dihydroxy-2-methyl-prop-2-yl)urea, N-(1,3-dihydroxy-2-hydroxymethyl-prop-2-yl)urea, and mixtures thereof; and
(b) at least one film-forming amino-silicone polymer selected from Polysilicone-35, Polysilicone-33, Polysilicone-29, and mixtures thereof; and

(ii) allowing the composition to remain in contact with the keratin fibers for a period ranging from about 5 seconds to about 96 hours.

In one embodiment, in the method described herein, in step (ii) the cosmetic composition is left in contact with the keratin fibers for a period ranging from about 5 seconds to about 96 hours, or from about 10 seconds to about 72 hours, or from about 10 seconds to about 48 hours.

In an embodiment, in the method described herein, in step (ii) the composition is allowed to remain in contact with the keratin fibers for a period ranging from about 10 seconds to until the keratin fibers are dry.

In an embodiment, in the method described herein, in step (ii) the keratin fibers are air-dried, towel-dried, blow-dried, heat-dried, steam-dried, radiation-dried or dried by combining two or more drying methods.

The term "air-dried" refers to letting the keratin fibers dry naturally by contact with air.

The term "towel-dried" refers to using a towel to dry keratin fibers, so the fibers can dry faster, in particular, using a towel to squeeze water out from the fibers.

The term "blow-dried" refers to using a blow dryer to dry the keratin fibers.

The term "heat-dried" refers to using a heat source using elevated temperatures to dry the keratin fibers, such as

flat ironing. In particular by using temperatures ranging from about 50°C to about 230°C, or from about 90°C to 200°C.

The term "steam-dried" refers to using a hot steam to dry the keratin fibers.

The term "radiation-dried" refers to using a radiation source, such as an Infrared Light source, to dry the keratin fibers.

In an embodiment, in the method described herein, in step (ii) the keratin fibers are dried by combining two or more drying methods.

In an embodiment, in the method described herein, in step (ii) the keratin fibers are towel-dried and, subsequently, are air-dried.

In an embodiment, in the method described herein, in step (ii) the keratin fibers are heat-dried and, simultaneously or subsequently, are steam-dried, in particular by using a steam iron.

In an embodiment, in the method described herein, in step (ii) the keratin fibers are steam-dried and, simultaneously or subsequently, are heated-dried.

In particular by using a steam iron, such as the steam iron commercially available from L'Oréal Professionnel under the trademark SteamPod.

In an embodiment, in the method described herein, in step (ii) the keratin fibers are blow-dried and, simultaneously, are radiation-dried.

In particular, by using a hair dryer commercially available from Zuvi Inc under the trademark Zuvi Halo Hair Dryer, or the hair dryer commercially available from L'Oréal Professionnel under the trademark AirLight Pro. Both hair driers combine an airflow with a ration source (infrared lamp).

In an embodiment, the method described herein additionally comprises the step (iii) removing the cosmetic composition from the keratin fibers by rinsing with water or by washing the keratin fibers with a shampoo.

In an embodiment, the method described herein additionally comprises the step (iiib) removing the cosmetic composition from the keratin fibers by washing the keratin fibers with a shampoo.

In an embodiment, the method described herein additionally comprises the step (iv) rinsing the keratin fibers with water.

In an embodiment, it is described a method for increasing the volume of keratin fibers comprising the steps of:

(i) treating the keratin fibers by topical application of a cosmetic composition comprising, in a cosmetically acceptable medium:

(a) at least urea and/or an urea derivative selected from N,N-dimethylurea, N-(2-hydroxyethyl)urea, N-(3-hydroxypropyl)urea, N-(2-hydroxypropyl)urea, N-(2-hydroxy-2-methyl-prop-2-yl)urea,N-(1,3-dihydroxy-2-methyl-prop-2-yl)urea, N-(1,3-dihydroxy-2-hydroxymethyl-prop-2-yl)urea, and mixtures thereof; and
(b) at least one film-forming amino-silicone polymer selected from Polysilicone-35, Polysilicone-33, Polysilicone-29, and mixtures thereof;

(ii) allowing the composition to remain in contact with the keratin fibers for a period ranging from about 5 seconds to about 96 hours; and
(iii) removing the cosmetic composition from the keratin fibers by rinsing with water or by washing the keratin fibers with a shampoo.

In an embodiment, it is described a method for increasing the volume of keratin fibers comprising the steps of:

(i) treating the keratin fibers by topical application of a cosmetic composition comprising, in a cosmetically acceptable medium:

(a) at least urea and/or an urea derivative selected from N,N-dimethylurea, N-(2-hydroxyethyl)urea, N-(3-hydroxypropyl)urea, N-(2-hydroxypropyl)urea, N-(2-hydroxy-2-methyl-prop-2-yl)urea,N-(1,3-dihydroxy-2-methyl-prop-2-yl)urea, N-(1,3-dihydroxy-2-hydroxymethyl-prop-2-yl)urea, and mixtures thereof; and
(b) at least one film-forming amino-silicone polymer selected from Polysilicone-35, Polysilicone-33, Polysilicone-29, and mixtures thereof;

(ii) allowing the composition to remain in contact with the keratin fibers for a period ranging from about 10 seconds to until the keratin fibers are dry; and
(iii) removing the cosmetic composition from the keratin fibers by rinsing with water or by washing the keratin fibers with a shampoo.

In an embodiment, it is described a method for increasing the volume of keratin fibers comprising the steps of:

(i) treating the keratin fibers by topical application of a cosmetic composition comprising, in a cosmetically acceptable medium:

(a) at least urea and/or an urea derivative selected from N,N-dimethylurea, N-(2-hydroxyethyl)urea, N-(3-hydroxypropyl)urea, N-(2-hydroxypropyl)urea, N-(2-hydroxy-2-methyl-prop-2-yl)urea,N-(1,3-dihydroxy-2-methyl-prop-2-yl)urea, N-(1,3-dihydroxy-2-hydroxymethyl-prop-2-yl)urea, and mixtures thereof; and
(b) at least one film-forming amino-silicone polymer selected from Polysilicone-35, Polysilicone-33, Polysilicone-29, and mixtures thereof;

(ii) allowing the composition to remain in contact with the keratin fibers for a period ranging from about 5 seconds to about 96 hours;
(iiib) removing the cosmetic composition from the keratin fibers washing the keratin fibers with a shampoo; and
(iv) rinsing the keratin fibers with water.

In an embodiment, it is described a method for increasing the volume of keratin fibers comprising the steps of:

(i) treating the keratin fibers by topical application of a cosmetic composition comprising, in a cosmetically acceptable medium:

(a) at least urea and/or an urea derivative selected from N,N-dimethylurea, N-(2-hydroxyethyl)urea, N-(3-hydroxypropyl)urea, N-(2-hydroxypropyl)urea, N-(2-hydroxy-2-methyl-prop-2-yl)urea,N-(1,3-dihydroxy-2-methyl-prop-2-yl)urea, N-(1,3-dihydroxy-2-hydroxymethyl-prop-2-yl)urea, and mixtures thereof; and
(b) at least one film-forming amino-silicone polymer selected from Polysilicone-35, Polysilicone-33, Polysilicone-29, and mixtures thereof;

(ii) allowing the composition to remain in contact with the keratin fibers for a period ranging from about 10 seconds to until the keratin fibers are dry;
(iiib) removing the cosmetic composition from the keratin fibers washing the keratin fibers with a shampoo; and
(iv) rinsing the keratin fibers with water.

In an embodiment, the cosmetic use of a cosmetic composition as defined above to increase the volume of keratin fibers is disclosed.
In an embodiment, it is disclosed the cosmetic use of a composition comprising, in a cosmetically acceptable medium:

(c) at least urea and/or an urea derivative selected from N,N-dimethylurea, N-(2-hydroxyethyl)urea, N-(3-hydroxypropyl)urea, N-(2-hydroxypropyl)urea, N-(2-hydroxy-2-methyl-prop-2-yl)urea, N-(1,3-dihydroxy-2-methyl-prop-2-yl)urea, N-(1,3-dihydroxy-2-hydroxymethyl-prop-2-yl)urea, and mixtures thereof; and
(d) at least one film-forming amino-silicone polymer selected from Polysilicone-35, Polysilicone-33, Polysilicone-29, and mixtures thereof;

for increasing the volume of keratin fibers.
The following examples are given in order to provide a person skilled in the art with a sufficiently clear and complete explanation of the present disclosure but should not be considered as limiting of the essential aspects of its subject, as set out in the preceding portions of this description.
Ingredients are identified by chemical or INCI name, or otherwise defined below.

## EXAMPLES

1. Preparation of the compositions

The compositions as shown in Table 1 were prepared by mixing and stirring until complete homogenization in a kettle. Composition C1 is a composition according to the present disclosure. Compositions CC1 and CC2 are comparative compositions.

Table 1 - Compositions (wt.% as active matter based on the total weight of the composition)

| Ingredients (INCI name) | Compositions | | |
|---|---|---|---|
| | C1 | CC1 | CC2 |
| Urea | 3 | 3 | --- |
| Polysilicone-29 | 0.2 | --- | 0.2 |
| Aqua (Water (Eau)) | Up to 100 | Up to 100 | Up to 100 |

2. Application of the compositions to keratin fibers

2.1. Pre-wash

Strands of virgin dark Caucasian hair were selected, each one approximately 22 cm long.

24 hours prior to the treatment, the strands were washed with a shampoo and rinsed under tap water according to the following process:

- the strands were soaked for 1 hour in a solution of 10 wt.% Pro You™ Neutral Shampoo (commercially available from Revlon Professional) and tap water;
- the strands were rinsed for 60 seconds under tap water;
- excess water was removed with a towel; and
- finally, the strands were left to dry naturally (air-dried) at room temperature (about 25°C).

2.2. Damaging (bleaching) hair

The strands of hair of step 2.1 were damaged with a bleach mark to be used as reference for measuring purposes, the bleach mark being located at the same position (approximately at 1/3 of the hair strands) according to the following process:

- a powder bleaching composition (Pro You™ The Lifter bleaching powder commercially available from Revlon Professional) was mixed with a 9% by weight aqueous hydrogen peroxide composition (Pro You™ The Developer commercially available from Revlon Professional) in a non-metallic bowl at a ratio 1:2 (powder:hydrogen peroxide);
- the strands were soaked with a brush with the mixture prepared above;
- the strands were wrapped in aluminum foil and were allowed to stand in an oven at 37°C for a period of 45 minutes;
- the strands were rinsed for 2-3 minutes under tap water;
- the strands were soaked for 30 seconds in a solution of 10 wt.% Pro You™ Neutral Shampoo (commercially available from Revlon Professional) and tap water, under agitation (about 950 rpm);
- the strands were rinsed for 30 seconds under tap water;
- excess water was removed with a towel; and
- finally, the strands were dried for 6 minutes using a Pro Iron Dryer 3000 City manufactured by Tectools and, after that, left to dry naturally (air-dried) at room temperature (about 25°C).

2.3. Application

The composition C1 and the comparative compositions CC1-CC2 of Ex. 1 were applied to the strands damaged as described in point 2.2 above, according to the following process:

2.3.1. Treatment 1: No rinse
3 locks of hair from each strand (approximately 2 g each lock) were selected.

One lock of hair were immersed for 10 minutes in one of the following solutions:

Composition C1
Comparative Composition CC1
Comparative Composition CC2

All the hair locks were combed to untangle them and to spread the compositions uniformly.

The hair locks were not rinsed and left to dry naturally (air-dried) overnight at room temperature (about 25°C).

The hair fiber diameter before and after the treatment was measured. For every strand, the diameter of 20 hair fibers was measured using a Nikon Eclipse E200 (40x objective) optical microscope attached to the digital camera ToupTek E3ISPM Series (CMOS USB3.0) equipped with ToupView software. Each fiber was measured three times.

2.3.2. Treatment 2: Shampooing

48 h later, the hair strands treated in point 2.3.1 above were subsequently shampooed as follows:

- the strands were soaked for 30 seconds in a solution of 10 wt.% Pro You™ Neutral Shampoo (commercially available from Revlon Professional) and tap water, under agitation (about 950 rpm);
- the strands were rinsed for 30 seconds under tap water;
- excess water was removed with a towel; and
- finally, the strands were left to dry naturally (air-dried) at room temperature (about 25°C).

The hair fiber diameter before and after the treatment was measured. For every strand, the diameter of 20 hair fibers was measured using a Nikon Eclipse E200 (40x objective) optical microscope attached to the digital camera ToupTek E3ISPM Series (CMOS USB3.0) equipped with ToupView software. Each fiber was measured three times.

The hair volume increase, expressed as hair diameter increase, was calculated as follows:

$$\%\ \text{Increase} = \frac{\text{diameter (after shampooing) - diameter (before shampooing)}}{\text{diameter (before shampooing)}} \times 100$$

The results are shown in Table 2. Each value represents the mean of 3 hair strands.

Table 2 - % Increase in the hair fiber diameter (shampooing step 48 h after the initial treatment).

| Compositions | % diameter increase |
|---|---|
| C1 (urea + Polysilicone-29) | 13.5 |
| CC1 (urea) | 8.0 |
| CC2 (Polysilicone-29) | 2.5 |

From the experimental results, it can be concluded that compositions of the present disclosure (Composition C1) are effective in producing noticeable increases in hair volume (expressed as hair diameter) that is maintained after a shampooing step.

2.4. Loss of hair volume due to the shampooing step

As indicated above, the hair volume increase, expressed as hair diameter increase, was measured in Treatment 1 (No rinse) and in Treatment 2 (Shampooing).

The ability of the compositions of the present disclosure to maintain the initial increase in hair volume even after a shampooing effect, i.e. the loss of the volumizing effect (loss of hair diameter) due to the shampooing step was calculated as follows:

$$\%\ \text{Loss of volume} = \frac{\text{\% diameter (after Treatment 1) - \% diameter (after Treatment 2)}}{\text{\% diameter (after Treatment 1)}} \times 100$$

The results are shown in Table 3.

Table 3 - % Loss of hair volume (hair fiber diameter) due to the shampooing step

| Compositions | % Loss |
|---|---|
| C1 (urea + Polysilicone-29) | 43 |
| CC1 (urea) | 48 |

(continued)

| Compositions | % Loss |
|---|---|
| CC2 (Polysilicone-29) | 65 |

From the experimental results, it can be concluded that compositions of the present disclosure (Composition C1) are effective in retaining the increase in hair volume (expressed as hair diameter) even after a shampooing step.

Modifications, which do not affect, alter, change or modify the essential aspects of the compositions and methods described above, are included within the scope of the present disclosure.

## Claims

**1.** A method for increasing the volume of keratin fibers comprising the steps of:

(i) treating the keratin fibers by topical application of a cosmetic composition comprising, in a cosmetically acceptable medium:

(a) at least urea and/or an urea derivative selected from N,N-dimethylurea, N-(2-hydroxyethyl)urea, N-(3-hydroxypropyl)urea, N-(2-hydroxypropyl)urea, N-(2-hydroxy-2-methyl-prop-2-yl)urea, N-(1,3-dihydroxy-2-methyl-prop-2-yl)urea, N-(1,3-dihydroxy-2-hydroxymethyl-prop-2-yl)urea, and mixtures thereof; and
(b) at least one film-forming amino-silicone polymer selected from Polysilicone-35, Polysilicone-33, Polysilicone-29, and mixtures thereof; and

(ii) allowing the composition to remain in contact with the keratin fibers for a period ranging from about 5 seconds to about 96 hours.

**2.** The method according to claim 1, wherein in step (ii) the composition is allowed to remain in contact with the keratin fibers for a period ranging from about 10 seconds to until the keratin fibers are dry.

**3.** The method according to claim 1 or claim 2, wherein in the cosmetic composition the component (a) is selected from urea, N,N-dimethylurea, N-(2-hydroxyethyl)urea and mixtures thereof.

**4.** The method according to any one of claims 1 to 3, wherein in the cosmetic composition the component (a) comprises urea.

**5.** The method according to any one of claims 1 to 4, wherein in the cosmetic composition the component (b) comprises Polysilicone-29.

**6.** The method according to any one of claims 1 to 6, wherein in the cosmetic composition the total amount of the component (a) is in the range of about 0.5 to about 10 wt.%, based on the total weight of the composition.

**7.** The method according to any one of claims 1 to 6, wherein in the cosmetic composition the total amount of the component (b) is in the range of about 0.05 to about 10 wt.%, based on the total weight of the composition.

**8.** The method according to claim 1, wherein the cosmetic composition comprises, in a cosmetically acceptable medium:

(a) urea, N,N-dimethylurea, N-(2-hydroxyethyl)urea and mixtures thereof; and
(b) at least one film-forming amino-silicone polymer selected from Polysilicone-35, Polysilicone-33, Polysilicone-29, and mixtures thereof.

**9.** The method according to claim 8, wherein the cosmetic composition comprises, in a cosmetically acceptable medium, based on the total weight of the composition:

(a) about 0.5-10 wt.% of urea, N,N-dimethylurea, N-(2-hydroxyethyl)urea and mixtures thereof; and
(b) about 0.05-10 wt.% of at least one film-forming amino-silicone polymer selected from Polysilicone-35, Polysilicone-33, Polysilicone-29, and mixtures thereof.

**10.** The method according to claim 8, wherein the cosmetic composition comprises, in a cosmetically acceptable medium:

(a) urea; and
(b) Polysilicone-29.

**11.** The method according to claim 10, wherein the cosmetic composition comprises, in a cosmetically acceptable medium, based on the total weight of the composition.

(a) about 0.5-10 wt.% of urea; and
(b) about 0.05-10 wt.% of Polysilicone-29.

**12.** The method according to any one of claims 1 to 11, wherein the keratin fibers are human hair.

**13.** The method according to any one of claims 1 to 12, wherein the cosmetic composition has a pH in the range from about 3.5 to about 8.5.

**14.** The method according to any one of claims 2 to 13, wherein in step (ii) the keratin fibers are air-dried, towel-dried, blow-dried, heat-dried, steam-dried, radiation-dried or dried by combining two or more drying methods.

**15.** The method according to any one of claims 1 to 14, additionally comprising the step (iii) removing the cosmetic composition from the keratin fibers by rinsing with water or by washing the keratin fibers with a shampoo.

Europäisches Patentamt
European Patent Office
Office européen des brevets

# EUROPEAN SEARCH REPORT

Application Number
EP 24 20 3826

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| Y | US 2020/375876 A1 (ARORA SHILPA [US] ET AL) 3 December 2020 (2020-12-03)<br>* page 1, paragraph 8-14 *<br>* page 2, paragraph 24 *<br>* composition 3;<br>pages 16-17; table 3 *<br>* pages 17-18; examples 4-5 *<br>----- | 1-15 | INV.<br>A61K8/42<br>A61K8/898<br>A61Q5/00<br>A61Q5/12 |
| Y | Anonymous: "Treatment Thick, Unruly Hair",<br>Mintel,<br>1 July 2022 (2022-07-01), pages 1-3, XP093254246,<br>* the whole document *<br>----- | 1-15 | |
| Y | FR 3 046 059 A1 (OREAL [FR])<br>30 June 2017 (2017-06-30)<br>* page 24; claim 1 *<br>* pages 26-27; claim 4 *<br>* page 30; claims 12-14 *<br>----- | 1-15 | |

TECHNICAL FIELDS SEARCHED (IPC)

A61Q
A61K

The present search report has been drawn up for all claims

1

| Place of search | Date of completion of the search | Examiner |
|---|---|---|
| The Hague | 28 February 2025 | Raposo, Antonio |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

EPO FORM 1503 03.82 (P04C01)

## ANNEX TO THE EUROPEAN SEARCH REPORT ON EUROPEAN PATENT APPLICATION NO. EP 24 20 3826

This annex lists the patent family members relating to the patent documents cited in the above-mentioned European search report.
The members are as contained in the European Patent Office EDP file on
The European Patent Office is in no way liable for these particulars which are merely given for the purpose of information.

28-02-2025

| Patent document cited in search report | Publication date | Patent family member(s) | Publication date |
|---|---|---|---|
| US 2020375876 A1 | 03-12-2020 | NONE | |
| FR 3046059 A1 | 30-06-2017 | NONE | |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- EC 12232009


### Non-patent literature cited in the description


- Illustrative list by category of cosmetic products. Council Directive of the European Communities No. 76/768/EEC. 27 July 1976
- International Cosmetic Ingredient Dictionary and Handbook. Personal Care Products Council (PCPC)
- *CHEMICAL ABSTRACTS*, 57-13-6
- *CHEMICAL ABSTRACTS*, 96-31-1
- *CHEMICAL ABSTRACTS*, 1320-51-0
- CTFA dictionary. 2004
- **THOMAS CLAUSEN et al.** Hair Preparations. *Ullmann's Encyclopedia of Industrial Chemistry*, 2006